(19) Europäisches Patentamt
European Patent Office
Office européen des brevets

(11) EP 0 780 132 B1

(12) **EUROPEAN PATENT SPECIFICATION**

(45) Date of publication and mention of the grant of the patent:
**24.07.2002 Bulletin 2002/30**

(51) Int Cl.[7]: **A61L 9/01**, A01K 1/015

(21) Application number: **96308617.8**

(22) Date of filing: **28.11.1996**

(54) **Malodor counteractant composition and process for using same**

Zusammensetzung zur Vernichtung von schlechten Gerüchen und Verfahren zu ihrer Verwendung

Composition détruisant les mauvaises odeurs et procédé d'utilisation

(84) Designated Contracting States:
**DE FR GB IT**

(30) Priority: **21.12.1995 US 8979**
**09.05.1996 US 647243**

(43) Date of publication of application:
**25.06.1997 Bulletin 1997/26**

(73) Proprietor: **INTERNATIONAL FLAVORS & FRAGRANCES INC.**
**New York New York 10019 (US)**

(72) Inventors:
- **Gordon, Mary E.**
  **County of Monmouth, New Jersey 07718 (US)**
- **Schreck, Lisa**
  **County of Monmouth, New Jersey 07724 (US)**
- **Hanna, Marie R.**
  **County of Monmouth,New Jersey 07735 (US)**
- **Sutcliffe, Ruth M.**
  **New Jersey, 07748 (US)**
- **Hamilton, William L**
  **New Jersey, 07748 (US)**

(74) Representative: **Brown, John David et al**
**FORRESTER & BOEHMERT**
**Pettenkoferstrasse 20-22**
**80336 München (DE)**

(56) References cited:

| | |
|---|---|
| **EP-A- 0 404 470** | **FR-A- 2 666 510** |
| **GB-A- 748 301** | **US-E- R E27 332** |

- **PATENT ABSTRACTS OF JAPAN vol. 016, no. 128 (C-0924), 2 April 1992 & JP 03 294219 A (T HASEGAWA CO LTD), 25 December 1991,**
- **PATENT ABSTRACTS OF JAPAN vol. 012, no. 198 (C-502), 8 June 1988 & JP 63 000266 A (TAKASAGO CORP), 5 January 1988,**
- **DATABASE WPI Section Ch, Week 8204 Derwent Publications Ltd., London, GB; Class D23, AN 82-07542E XP002027437 & SU 821 483 A (TASHK PERFUM COSMET) , 15 April 1981**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 780 132 B1

## Description

Our invention is directed to a malodor counteractant composition and a process of using same.

Our malodor composition covers the following malodors, inter alia:

pet (cat urine);
human urine;
human fecal;
smoking tobacco;
cooking (seafood);
cooking (onion) ;
cooking (fried chicken);
garbage; and
mildew.

The malodor maskant composition of our invention contains from 20% up to 60% of a musk note which is GALAXOLIDE®; from 30% up to 70% of a citrus note which is CITRAL (TM); and from 1% up to 20% of a mint note which is corn mint oil.

The malodor maskant composition of our invention is intended to be used at levels of from 0.5% by weight up to 20% by weight based on the total quantity of composition containing fragrance and maskant.

The final fragrance maskant composition is intended to be incorporated into consumer products (e.g., air, carpet and upholstery fresheners) at levels of from about 0.6% up to about 25% by weight of the consumer product.

The musk note of the invention is GALAXOLIDE® (registered trademark of International Flavors & Fragrances Inc. of New York, New York). Examples of other musk notes include pentadecanalide, TONALIDE®, EXALTOLIDE® (trademark of Firmenich, etc. of Geneva, Switzerland) and the like.

The citrus note of the invention is citral. Examples of other citrus notes include lemon oil, orange oil, geranylnitrile, citronellol and the like.

The mint note of the invention is corn mint oil. Examples of other mint notes include menthone, menthol, and the like.

The musk note of the invention is GALAXOLIDE®, the nature of which is set forth on pages 9 and 10, infra. The preferred citrus note is citral refined; and the mint note of the invention is corn mint oil.

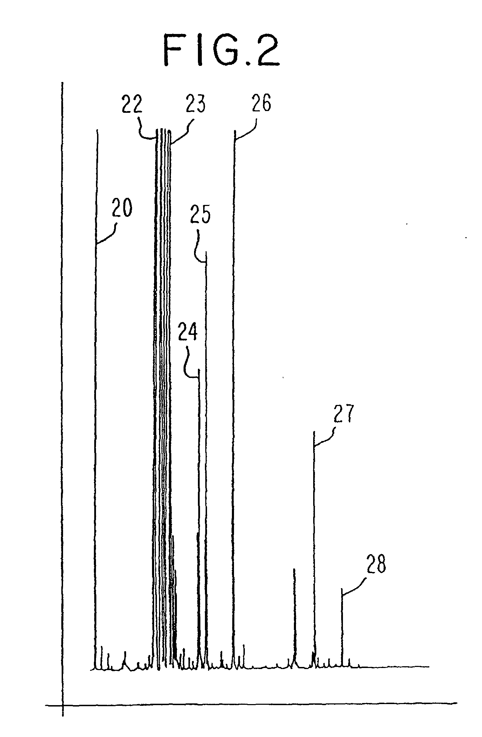

The corn mint oil is defined according to Figures 1 and 2.

Figure 1 is a GLC profile for the corn mint oil used in the preferred embodiment of our invention (conditions: 15 meter carbowax-20M column programmed from 70-220°C at 4°C per minute).

Figure 2 is another GLC profile of the corn mint oil used in the preferred embodiment of our invention (conditions: 50 meter OV1, dual fused silica system programmed from 70-220°C at 4°C per minute).

The most preferred embodiment is:

40% GALAXOLIDE®;
50% citral refined; and
10% corn mint oil.

Malodor neutralization is evaluated by placing a specified amount of malodor into an uncapped, 28 g (1 ounce) wide-mouth jar. This jar is placed into an 227 g (8 ounce) wide-mouth jar containing a specified amount of test material or finished fragrance. The quantities of malodor and fragrance are dependent on the specific malodor model used. The 227 g (8 ounce) jar is capped and allowed to equilibrate. The panelists then rate the headspace for (1) total intensity, (2) malodor intensity, (3) overall like/dislike (hedonics). They are provided with an identified sample of malodor as a reference. Each group of samples presented to the panel contains an unidentified positive control (a fragrance material with no malodor), two unidentified negative controls (malodor with no fragrance) and up to twelve test samples (each fragrance is presented in duplicate or triplicate).

It is to be noted that evaluations are carried out by 18-24 panelists who have been extensively screened for their olfactory acuity and trained in the method of evaluation, Magnitude Estimation. Magnitude Estimation of odor intensities provides normally distributed data that can be analyzed by parametric statistical techniques. For information on Magnitude Estimation, see ASTM Document #E1697-95, *Standard Test Method for Unipolar Magnitude Estimation of Sensory Attributes,* available from ASTM, 100 Barr Harbor Drive, West Conshohocken, Pennsylvania 19428-2959 (U.S.A.). Panelist performance is constantly monitored and reviewed.

All samples are coded with random 5-digit numbers. Panelists are instructed to select samples from the test set and evaluate them in a random order.

The results of these experiments are expressed in terms of malodor reduction (neutralization), change in overall intensity and improvement in hedonics. The percent malodor reduction is calculated as:

% Neutralization = 100 x [1-(malodor intensity/total intensity)].

The positive control is normally rated as providing approximately a 95% malodor neutralization; the negative control is less than 10%.

One of the important attributes of the formulations of our invention is that their efficacy should not be achieved by "overpowering" the malodor but by "blending" with it. In order to substantiate this attribute, the total intensity of the "fragrance + malodor" sample cannot be statistically more intense than the "malodor" sample alone. Also, there must be a statistically significant improvement in the perceived pleasantness (hedonics). Regarding the foregoing, reference is herewith made to Example C, infra.

The technology which we have developed as exemplified below and as set forth, supra, is based upon a fragrance composition's ability to blend with a specific malodor in such a fashion as to be quantified and documented. In addition to having the appropriate aesthetic appeal, these fragrances must also meet the following technical criteria:

1. significant reduction in perceived malodor when compared to appropriate control fragrances;

2. no significant increase in overall intensity when the fragrance is combined with malodor; and

3. significant increase in the level of perceived pleasantness.

The following examples are intended to indicate how the invention is to be practiced, but the invention is not to be limited thereto and is only to be limited to the claims as set forth, infra.

**EXAMPLE 1**

EXAMPLE FRAGRANCE A: The following floral aldehydic fragrance was prepared.

| Ingredients | Reference Note | Parts |
|---|---|---|
| Amyl Cinn Ald Coeur | 1 | 0.0157 |
| Benz Sal | 2 | 0.0714 |
| Cinn Alc | 3 | 0.0143 |
| CYCLACET® | 4 | 0.0071 |
| CYCLAPROP® | 5 | 0.0071 |
| Dihydro Myrcenol | - | 0.0143 |
| Dihydro Terpineol | - | 0.0714 |
| Dipropylene Glycol | - | 0.0957 |
| GALAXOLIDE Dep 50 Pct | 6 | 0.1429 |
| Hexyl Cinn Ald | 7 | 0.1986 |
| LILIAL® | 8 | 0.1486 |
| LYRAL® | 9 | 0.0229 |
| Meth Ionone Gamma A | 10 | 0.0286 |
| Phen Eth Acet | 11 | 0.0286 |
| Phen Eth Alc White Extra | 12 | 0.1000 |
| TONALIDE® | 13 | 0.0214 |
| TRIMOFIX "O"® | 14 | 0.0071 |

## REFERENCE NOTES FOR FRAGRANCE A

1. ∝ Amyl cinnamic aldehyde
2. Benzyl salicylate
3. Cinnamyl alcohol
4. Hexahydro-4,7-methanoinden-5-yl acetate
5. Hexahydro-4,7-methanoinden-5-yl prepionate
6. Structures on pages 1 and 2, infra
7. ∝ Hexylcinnamic aldehyde
8. p-t-butyl-∝-methyl-dihydro cinnamic aldehyde
9. (4-hydroxy-4-methylpentyl)-3-cyclohexene-1-carboxaldehyde
10. γ-Methyl ionone
11. β-Phenyl ethyl acetate
12. β-Phenyl ethyl alcohol
13. 6-Acetyl-1,2,3,4-tetrahydro-1,1,2,4,4,7-hexamethyl naphthalene
14. Methyl-2,6,10-trimethyl-2,5,9-cyclododecatrien-1-yl ketone

This fragrance is used in the following Examples:

| Malodor Neutralization Test Results | | | |
|---|---|---|---|
| **Fragrance** | **% Malodor Neutralization (b)** | **% Chance in Overall Intensity (c)** | **% Improvement in Hedonic (d)** |
| A | 42 | 14 | 27 |

a. Results are based on the average of two independent samples, each evaluated by 18-24 panelists;
b. Percent neutralization + 100 x [1-malodor intensity/total intensity];
c. Percent difference between the overall intensity of the malodor sample and that of the fragranced sample. Negative numbers indicate an overall reduction in intensity. Those differences which are statistically significant ($p<0.05$, one-tailed t statistic) are indicated by "*"; and
d. Percent difference between the hedonic rating of the pure malodor sample and that of the fragranced sample. A 100% reduction would be the neutral point on the scale. Those differences which are statistically significant ($P<0.05$, one-tailed statistic) are indicated by "*".

The evaluations were carried out according to the procedure set forth on page 2, lines 23-30 and page 3, lines 1-22. The malodor used in the example is a synthetic cat urine composed of ingredients determined by NMR, IR and mass spectral analyses to be present in natural cat urine.

The following malodor maskant formulation is prepared and called the NEUTRIFF® formulation (trademark of International Flavors & Fragrances Inc.):

## EXAMPLE NEUTRIFF® FORMULATION

40% GALAXOLIDE® (50% solution in diethylphthalate);
50% CITRAL® refined;
10% Corn mint oil (having a GLC profile as set forth in Figures 1 and 2).

This NEUTRIFF® formulation is used in the following examples:

The following table sets forth the increase in percent pet malodor coverage as a result of adding the NEUTRIFF® formulation described above to the fragrance described above in a 90:10 ratio (fragrance:NEUTRIFF®).

The evaluations were carried out according to the procedure set forth on page 2, lines 23-30 and page 3, lines 1-22, supra. The malodor used in the example is a synthetic cat urine composed of ingredients determined by NMR, IR and mass spectral analyses to be present in natural cat urine.

In the following example, the "expected percent malodor coverage equals (concentration of original fragrance x percent malodor neutralization provided by the original fragrance) plus (concentration of NEUTRIFF® formulation x percent malodor neutralization provided by the NEUTRIFF®:

| NEUTRIFF® DATA | | | | | |
|---|---|---|---|---|---|
| **Fragrance ID** | **% Pet Malodor Neutralization by Fragrance without NEUTRIFF® (a)** | **% Pet Malodor Neutralization with NEUTRIFF® Alone (a)** | **Expected % Pet Malodor Neutralization of Combination of Fragrance with NEUTRIFF® (b)** | **Actual % Pet Malodor Neutralization of Combination of Fragrance with NEUTRIFF® (a)** | **% Increase in Pet Malodor Neutralization (c)** |
| A | 42 | 88 | 47 | 81 | 72 |

a. Percent neutralization = 100 x [1-malodor intensity/ total intensity];

b. Expected percent neutralization = (concentration of original fragrance x % neutralization provided by the original fragrance) + (concentration of NEUTRIFF® x % neutralization provided by the NEUTRIFF®; and

(c) Percent increase = (actual - expected) ÷ (expected). This is statistically significant $p<0.05$.

**EXAMPLE 2**

NEUTRIFF® formulation described in Example 1 was added to the following fragrances in a ratio of 90:10 (fragrance:NEUTRIFF®). The sensory evaluation technique and the calculation of the expected percent malodor neutralization are as described in the previous example.

| NEUTRIFF® DATA | | | | | |
|---|---|---|---|---|---|
| **Fragrance ID** | **% Pet Malodor Neutralization by Fragrance without NEUTRIFF® (a)** | **% Pet Malodor Neutralization with NEUTRIFF® Alone (a)** | **Expected % Pet Malodor Neutralization of Combination of Fragrance with NEUTRIFF® (b)** | **Actual % Pet Malodor Neutralization of Combination of Fragrance with NEUTRIFF® (a)** | **% Increase in Pet Malodor Neutralization (c)** |
| B | 61 | 88 | 64 | 83 | 30 |
| C | 61 | 88 | 64 | 80 | 25 |
| D | 61 | 88 | 64 | 77 | 20 |
| E | 62 | 88 | 65 | 79 | 22 |
| F | 64 | 88 | 66 | 80 | 21 |
| G | 44 | 88 | 48 | 84 | 75 |
| H | 62 | 88 | 65 | 83 | 28 |

a. Percent neutralization = 100 x [1-malodor intensity/ total intensity];

b. Expected percent neutralization - (concentration of original fragrance x % neutralization provided by the original fragrance) + (concentration of NEUTRIFF® x % neutralization provided by the NEUTRIFF®; and

(c) Percent increase = (actual - expected) ÷ (expected). This is statistically significant $p<0.05$.

Description of Fragrances B-H are as follows:

| Fragrance | Description |
|---|---|
| B | Floral, fruity, woody and musk |
| C | Citrus and herbaceous |
| D | Citrus, herbaceous and fougere fresh |
| E | Citrus, fruity, floral, woody and musk |
| F | Fruity, floral, woody and musk |
| G | Floral, amber and woody |

(continued)

| Fragrance | Description |
|---|---|
| H | Fresh herbal, woody and mint |

**CONCLUSION:**

The original fragrances (A through H) as described in Examples 1 and 2 were developed to be aesthetically appropriate for a specific household product application. Although these fragrances are hedonically acceptable, they do not provide an adequate level of malodor neutralization. The NEUTRIFF® formulation described herein provides adequate malodor neutralization, but lacks the aesthetic appeal and variety demonstrated by fragrances A through H. When the NEUTRIFF® is combined with any one of the example fragrances (A through H), an unexpected, unobvious and advantageous increase in malodor neutralization is achieved without significantly altering the hedonic impression. Furthermore, these combinations significantly improve the overall hedonics (of the test malodor) without significantly increasing the overall intensity.

The term GALAXOLIDE® is a mixture of compounds having the structures:

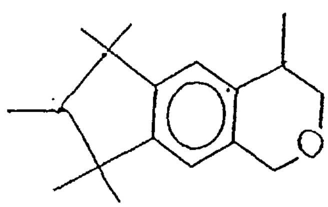 ; 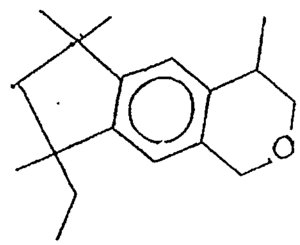 ;

and

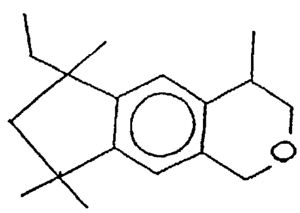

Other musks can be substituted for the GALAXOLIDE®, for example, those having the structures:

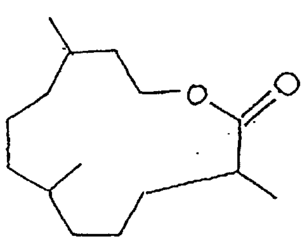 and 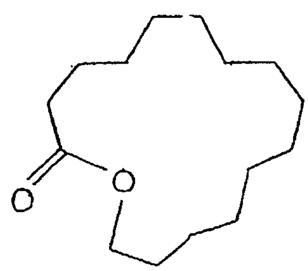

The citrus notes can be, for example, a mixture of 45-65% geranial having the structure:

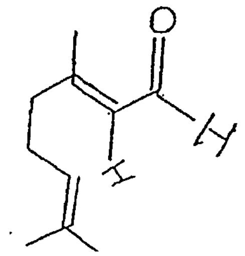

and 30-50% neral having the structure:

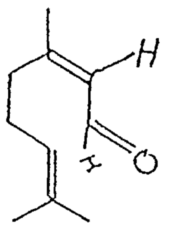

and such a mixture is also known as "CITRAL™".

**DETAILED DESCRIPTION OF THE DRAWINGS**

Referring to Figure 1, the GLC profile for corn mint oil, the peak indicated by reference numeral 10 is for α-pinene. The peak indicated by reference numeral 11 is for β-pinene. The peak indicated by reference numeral 12 is for sabinene. The peak indicated by reference numeral 13 is for limonene. The peak indicated by reference numeral 14 is for 3-octanol. The peak indicated by reference numeral 15 is for menthone. The peak indicated by reference numeral 16 is for isomenthone. The peak indicated by reference numeral 106 is for β-bourbonene. The peak indicated by reference numeral 17 is for menthol acetate. The peak indicated by reference numeral 18 is for neomenthol. The peak indicated by reference numeral 19 is for β-caryophyllene. The peak indicated by reference numeral 101 is for menthol. The peak indicated by reference numeral 102 is for a mixture of isomenthol and pulegone. The peak indicated by reference numeral 103 is for α-terpineol. The peak indicated by reference numeral 104 is for germacrene-D. The peak indicated by reference numeral 105 is for piperitone.

Referring to Figure 2, another GLC profile for corn mint oil, the peak indicated by reference numeral 20 is for limonene. The peak indicated by reference numeral 22 is for menthone. The peak indicated by reference numeral 23 is for menthol. The peak indicated by reference numeral 24 is for pulegone. The peak indicated by reference numeral 25 is for piperitone. The peak indicated by reference numeral 26 is for menthol acetate. The peak indicated by reference numeral 27 is for β-caryophyllene. The peak indicated by reference numeral 28 is for germacrene-D.

The features disclosed in the foregoing description, in the following claims and/or in the accompanying drawings, may both separately and in any combination thereof, be material for realising the invention in diverse forms thereof.

**Claims**

1. A composition of matter containing from 20% up to 60% by weight of a musk aroma material which is GALAXOLIDE ®, a mixture of compounds having the structures:

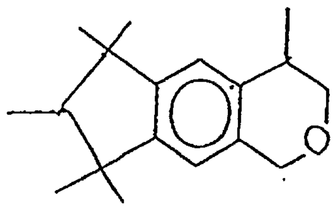 ; 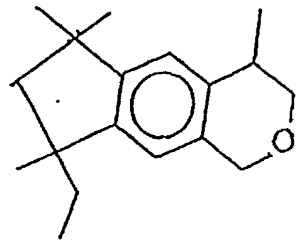 ;

and

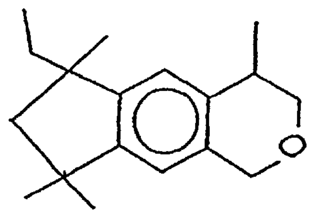

from 30% up to 70% by weight of a citrus aroma material which is CITRAL™, a mixture of 45%-65% geranial having the structure:

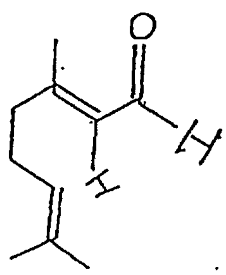

and 30-50% neral having the structure:

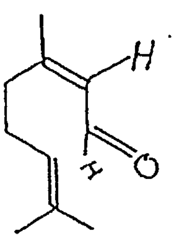

and from 1% up to 20% by weight of a mint aroma material which is corn mint oil.

**2.** The composition of Claim 1 containing 40% GALAXOLIDE®; 50% CITRAL™ refined; and 10% corn mint oil.

**3.** A process for masking malodor comprising the steps of:

(1) admixing the composition of Claim 1 or 2 with a compatible, fragrance formulation whereby the weight percent of the composition of Claim 1 or 2 in the resulting composition is from 0.5% up to 20%; and
(2) applying the resulting composition to a malodorous composition whereby the malodor thereof is masked.

4. A process for masking malodor comprising the step of applying the composition of claim 1 or 2 to a malodorous composition whereby the malodor thereof is masked.

5. The process of Claim 3 or 4 wherein the malodor masked is selected from the group consisting of:

pet (cat urine);
human urine;
human fecal;
smoking tobacco;
cooking;
garbage; and milden.

**Patentansprüche**

1. Chemische Zusammensetzung, enthaltend 20 bis 60 Gew.-% eines Moschusaromastoffes, welcher GALAXOLIDE® ist, ein Gemisch an Verbindungen mit den Strukturen:

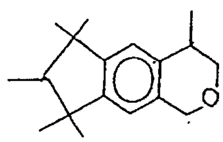

;

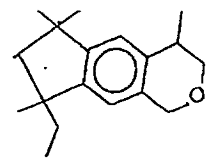

;

und

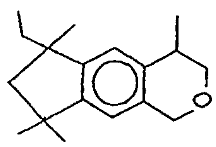

30 bis 60 Gew.-% eines Citrusaromastoffes, welcher CITRAL™ ist, ein Gemisch von 45% - 65% Geranial mit der Struktur:

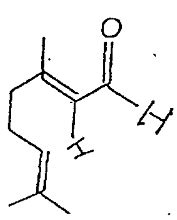

und 30 - 50% Neral mit der Struktur:

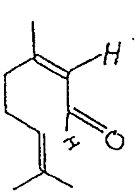

und 1 bis 20 Gew.-% eines Minzaromastoffes, welcher das Öl der gelben Minze ist.

**2.** Zusammensetzung nach Anspruch 1, enthaltend 40% GALAXOLIDE®; 50% raffiniertes CITRAL™; und 10% Öl der gelben Minze.

**3.** Verfahren zum Überdecken üblen Geruchs, die Schritte umfassend:

(1) der Zusammensetzung von Anspruch 1 oder 2 eine kompatible Duftformulierung beizumischen, wodurch die Gewichtsprozent der Zusammensetzung von Anspruch 1 oder 2 in der sich ergebenden Zusammensetzung 0,5% bis 20% betragen; und

(2) die sich ergebende Zusammensetzung einer übelriechenden Zusammensetzung zuzuführen, wodurch deren übler Geruch überdeckt wird.

**4.** Verfahren zum Überdecken üblen Geruchs, den Schritt umfassend, die Zusammensetzung von Anspruch 1 oder 2 einer übelriechenden Zusammensetzung zuzuführen, wodurch deren übler Geruch überdeckt wird.

**5.** Verfahren nach Anspruch 3 oder 4, bei dem der überdeckte üble Geruch aus der Gruppe ausgewählt ist, die besteht aus:

Haustiere (Katzenurin);
menschlicher Urin;
menschliche Fäkalien;
Rauchtabak;
Kochen;
Abfall; und
Schimmel.

**Revendications**

**1.** Composition de matière contenant entre 20 % et jusqu'à 60 % en poids d'une matière à odeur de musc qui est le GALAXOLIDE®, mélange de composés ayant les structures suivantes :

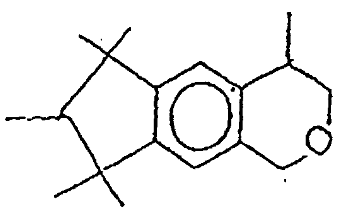 ; 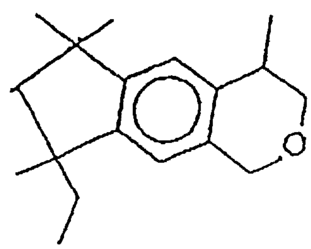 ;

et

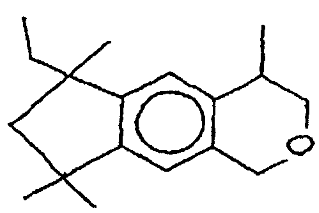

entre 30 % et jusqu'à 70 % en poids d'une matière à odeur d'agrume qui est le CITRAL™, mélange de 45 % à 65 % de géranial ayant la structure suivante :

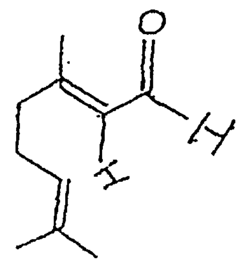

et de 30 % à 50 % en poids de néral ayant la structure suivante :

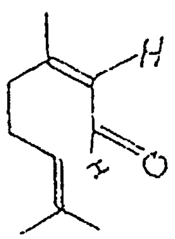

et entre 1 % jusqu'à 20 % en poids d'une matière à arôme de menthe qui est de l'huile de menthe des champs.

**2.** Composition selon la revendication 1, contenant 40 % de GALAXOLIDE®, 50 % de CITRAL™ raffiné ; et 10 % d'huile de menthe des champs.

**3.** Procédé permettant de masquer les mauvaises odeurs comprenant les étapes suivantes :

(1) ajouter à la composition suivant la revendication 1 ou 2, une formulation de fragrance compatible, où le pourcentage en poids de la composition selon la revendication 1 ou 2, dans la composition résultante, est compris entre 0,5 % et jusqu'à 20 % ; et

(2) appliquer la composition résultante à une composition ayant une mauvaise odeur, grâce à quoi la mauvaise odeur de celle-ci est masquée.

**4.** Procédé permettant de masquer les mauvaises odeurs comprenant l'étape consistant à appliquer la composition selon la revendication 1 ou 2 à une composition ayant une mauvaise odeur, grâce à quoi la mauvaise odeur en elle-même est masquée.

**5.** Procédé selon la revendication 3 ou 4, dans lequel la mauvaise odeur masquée est choisie parmi le groupe consistant en :

animal de compagnie (urine de chat) :
urine humaine ;
excréments humains ;
tabac ;
cuisine ;
poubelles ; et
moisissure

FIG.1

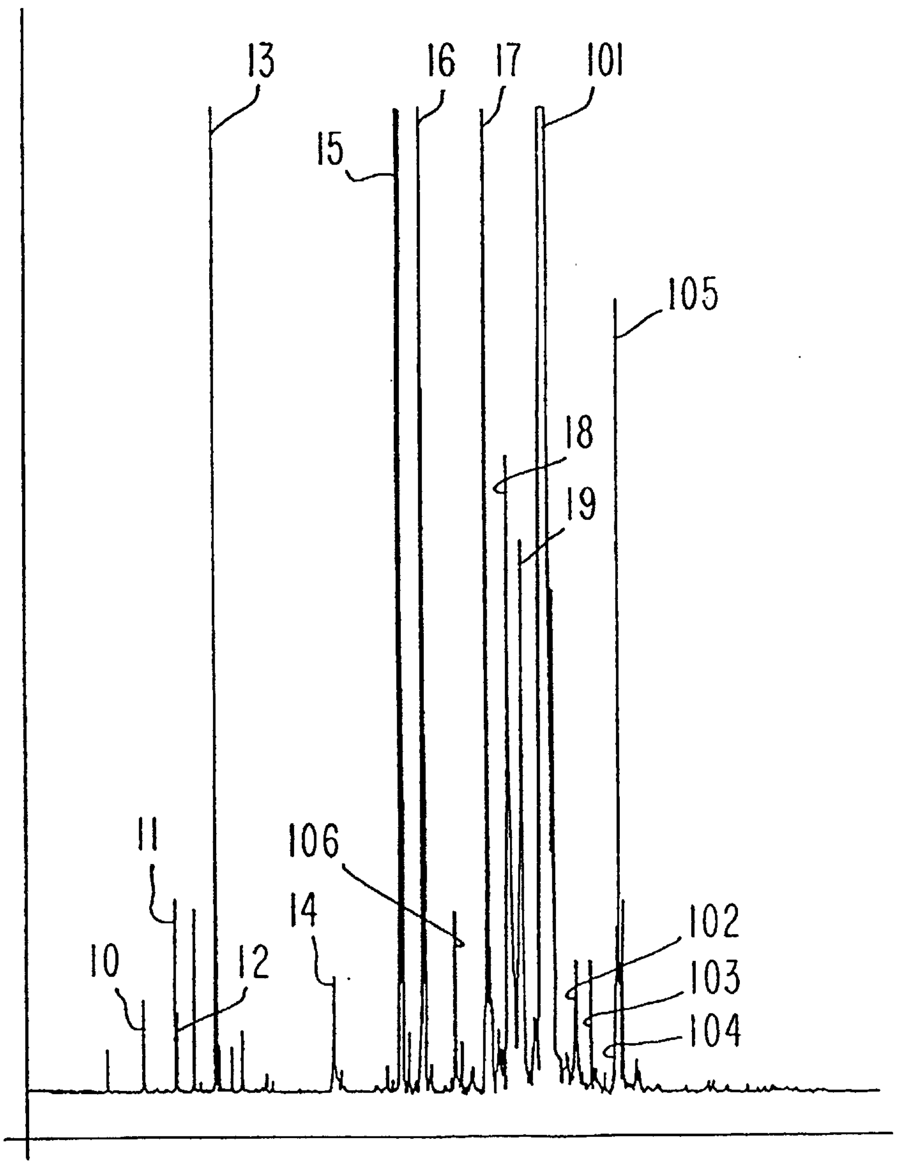

FIG.2